# EUROPEAN PATENT APPLICATION

(11) **EP 3 520 671 A1**
(43) Date of publication of application: **07.08.2019**
(21) Application number: 17855578.5
(22) Date of filing: 01.09.2017
(51) Int. Cl.: A61B 1/00

(54) **PROCESSOR DEVICE, ENDOSCOPE SYSTEM, AND METHOD FOR OPERATION OF PROCESSOR DEVICE**

(30) Priority: 30.09.2016 JP 2016192893
(71) Applicant: FUJIFILM Corporation, Tokyo 106-8620 (JP)
(72) Inventor: KAMON Shumpei, Ashigarakami-gun Kanagawa 258-8538 (JP)
(74) Representative: Klunker IP Patentanwälte PartG mbB
(86) International application number: PCT/JP2017/031642
(87) International publication number: WO 2018/061620

(57) **Abstract**

Provided are a processor device, an endoscope system, and a method of operating the processor device capable of distinguishing respective tissues included in an observation target. A Bs image signal, a Gs image signal, and an Rs image signal obtained by imaging the observation target including at least one tissue are acquired. A calculated image is obtained by performing specific calculation processing on the basis of these images of bands. The tissue discrimination unit 74 discriminates the tissue on the basis of a brightness value of the Bs image signal and the calculated image.

## Description

### BACKGROUND OF THE INVENTION

### 1. Field of the Invention

The present invention relates to a processor device, an endoscope system, and a method of operating a processor device, for displaying tissue included in an observation target.

### 2. Description of the Related Art

In the medical field, diagnosis using an endoscope system comprising a light source device, an endoscope, and a processor device has been performed widely. The endoscope system irradiates an observation target via the endoscope with illumination light from the light source device, and the processor device produces an image of the observation target on the basis of an image signal obtained by capturing the observation target under illumination with the illumination light. By displaying the image on a monitor, a doctor can perform diagnosis while viewing this image on the monitor.

Additionally, in recent years, diagnosing a lesion has also been performed by paying attention to the structure of tissue, such as a blood vessel structure and a glandular structure. In a case where observation is performed by paying attention to the structure of the tissue in this way, for example, as shown in WO2012/147505, feature amounts of various structures, such as a blood vessel feature amount, are extracted from an image obtained by imaging the observation target, and images according to structure are generated and displayed on the basis of the extracted feature amounts. Additionally, in JP2015-062728, surface layer blood vessels and middle-depth layer blood vessels are extracted from the image obtained by imaging the observation target and displayed.

### SUMMARY OF THE INVENTION

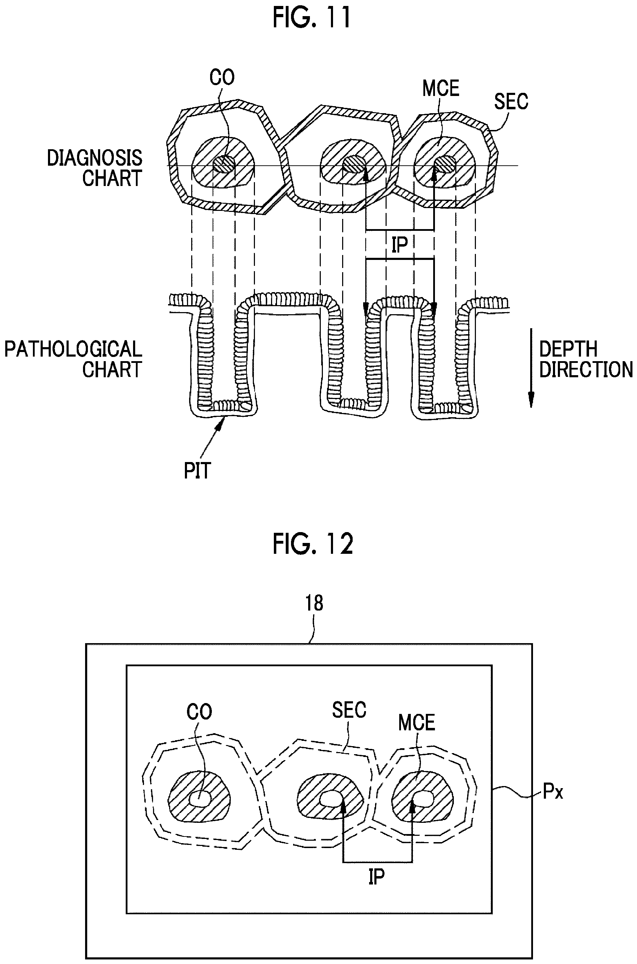

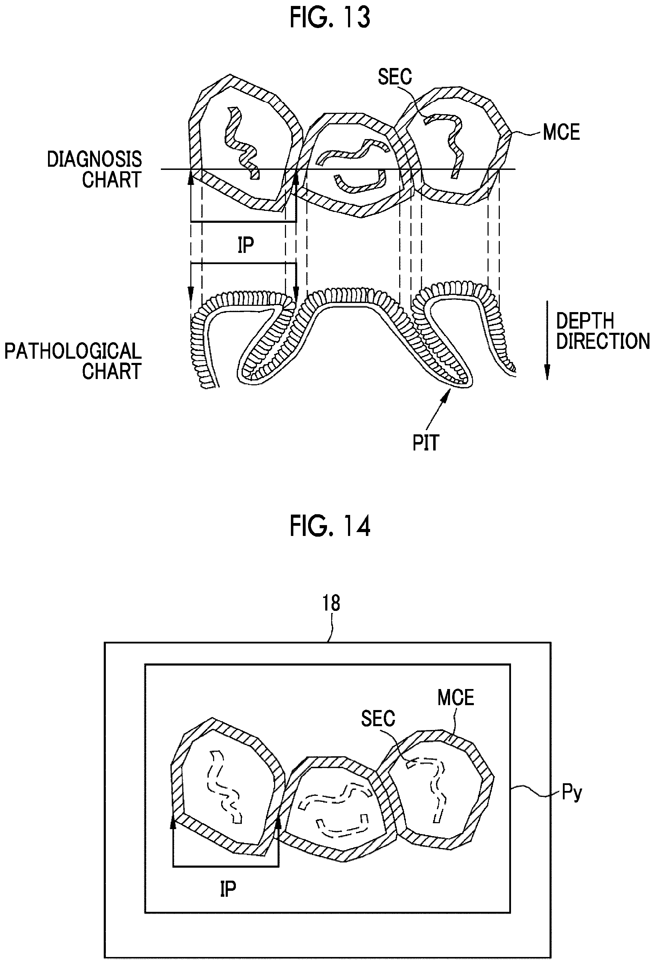

A plurality of structures, such as the blood vessel structure and the glandular structure, are present as structures useful for the diagnosis of the lesion, and these structures are respectively different from each other in shape, brightness, and the like. Additionally, the shape, brightness, and the like of the structures vary widely depending on the degree of progress of the lesion. Moreover, even in a case where the brightness and the like are the same, completely different tissues are also present. For example, since a sub epithelial capillary SEC and an intervening part IP (refer to Figs. 11 and 13) that can be observed in a case where the stomach is magnified have the same light absorption characteristics, respectively, these are displayed with the same brightness. For that reason, it is difficult to discriminate the sub epithelial capillary SEC and the intervening part IP only with the brightness. From the above, distinguishing and displaying the respective tissues included in the observation target have been required.

An object of the invention is to provide a processor device, an endoscope system, and a method of operating the processor device capable of distinguishing respective tissues included in an observation target.

A processor device of the invention comprises an image acquisition unit that acquires images in a plurality of bands obtained by imaging an observation target including at least one tissue; a specific calculation processing unit that performs specific calculation processing on the basis of the images in the plurality of bands to obtain a calculated image; and a tissue discrimination unit that discriminates the tissue on the basis of a brightness value of at least one image of band among the images in the plurality of bands and the calculated image.

The processor device further comprises a brightness discrimination unit that discriminates a high-brightness region where the brightness value is equal to or more than a specific threshold value, and a low-brightness region where the brightness value is less than the specific threshold value, and the tissue discrimination unit discriminates the tissue among candidates of the tissue included in at least one of the high-brightness region or the low-brightness region in accordance with contents of the calculated image. An example of the specific calculation processing is shape discrimination processing for discriminating a shape of the tissue and the calculated image is an image subjected to the shape discrimination processing. In this case, it is preferable that the tissue discrimination unit discriminates the tissue in the low-brightness region in accordance with the shape. Additionally, another example of the specific calculation processing is difference processing for combining the images in the plurality of bands to calculate a difference between the images in the plurality of bands or ratio calculation processing for combining the images in the plurality of bands to calculate a ratio of the images in the plurality of bands and the calculated image is an image subjected to the difference processing or an image subjected to the ratio calculation processing. In this case, it is preferable that the tissue discrimination unit discriminates the tissue in the low-brightness region in accordance with the difference or the ratio. It is preferable that the tissue is a capillary, a crypt opening, an intervening part, a marginal crypt epithelium, an intra-epithelial papillary capillary loop (IPCL), or a dendritic blood vessel.

It is preferable that an endoscope system of the invention comprises a processor device of the invention described above; an individual-tissue image generation unit that generates an individual-tissue image distinguished and displayed for each tissue in accordance with a discrimination result of the tissue discrimination unit; and a display unit that displays the individual-tissue image. It is preferable that a specific tissue among the tissues is displayed in an enhanced manner on the individual-tissue image. It is preferable that the individual-tissue image is displayed in different colors for each tissue. It is preferable that a plurality of tissues are respectively displayed in a switched manner in accordance with specific display timings on the individual-tissue image.

A processor device of the invention comprises an image acquisition unit that acquires images in a plurality of bands obtained by imaging an observation target including at least one tissue; a brightness discrimination unit that discriminates a high-brightness region where a brightness value at least one image of band among the images in the plurality of bands is equal to or more than a specific threshold value, and a low-brightness region where the brightness value is less than the specific threshold value; a shape discrimination unit that performs shape discrimination processing for discriminating a shape of the tissue on the basis of the images in the plurality of bands; and a tissue discrimination unit that discriminates the tissue among candidates of the tissue included in at least one of the high-brightness region or the low-brightness region in accordance with the shape.

A method of operating a processor device of the invention comprises the steps of acquiring images in a plurality of bands obtained by imaging an observation target including at least one tissue, using an image acquisition unit; performing specific calculation processing on the basis of the images in the plurality of bands to obtain a calculated image, using a specific calculation processing unit; and discriminating the tissue on the basis of a brightness value of at least one image of band among the images in the plurality of bands and the calculated image, using a tissue discrimination unit.

According to the invention, it is possible to distinguish and display the respective tissues included in the observation target.

### BRIEF DESCRIPTION OF THE DRAWINGS

Fig. 1 is an external view of an endoscope system.
Fig. 2 is a block diagram illustrating the functions of the endoscope system of Embodiment 1A.
Fig. 3 is a graph illustrating the spectroscopic spectrum of violet light V, blue light B, blue light Bx, green light G, and red light R.
Fig. 4 is a graph illustrating the spectroscopic spectrum of normal light of Embodiment 1A.
Fig. 5 is a graph illustrating the spectroscopic spectrum of special light of Embodiment 1A.
Fig. 6 is a block diagram illustrating the functions of an individual-tissue image processing unit.
Fig. 7 is an explanatory view illustrating brightness discrimination processing.
Fig. 8 is an explanatory view illustrating shape discrimination processing.
Fig. 9 is an explanatory view illustrating tissue discrimination processing of Embodiment 1A.
Fig. 10 is an explanatory view illustrating that four types of images of a marginal crypt epithelium image, a crypt opening image, a blood vessel image, and an intervening part image are switched and displayed.
Fig. 11 is an explanatory view illustrating a diagnosis chart and a pathological chart of a normal stomach that is magnified using a zoom lens.
Fig. 12 is an image view illustrating an individual-tissue image in which the marginal crypt epithelium in the normal stomach is enhanced.
Fig. 13 is an explanatory view illustrating a diagnosis chart and a pathological chart of an irregular-structure stomach that is magnified using the zoom lens.
Fig. 14 is an image view illustrating an individual-tissue image in which the marginal crypt epithelium in the irregular-structure stomach is enhanced.
Fig. 15 is a flowchart illustrating an individual-tissue display mode.
Fig. 16 is a graph illustrating the spectrum of light to emit in Embodiment 1B.
Fig. 17 is a block diagram illustrating the functions of an individual-tissue image processing unit.
Fig. 18 is an explanatory view illustrating difference processing.
Fig. 19 is an explanatory view illustrating tissue discrimination processing of Embodiment 1B.
Fig. 20 is an explanatory view illustrating esophageal superficial vascular network.
Fig. 21 is an explanatory view illustrating shape discrimination processing of Embodiment 1C.
Fig. 22 is an explanatory view illustrating tissue discrimination processing of Embodiment 1C.
Fig. 23 is an explanatory view illustrating ratio calculation processing of Embodiment 1C.
Fig. 24 is an explanatory view illustrating another tissue discrimination processing of Embodiment 1C different from that in Fig. 22.
Fig. 25 is a block diagram illustrating the functions of an endoscope system of a second embodiment.
Fig. 26 is a graph illustrating the spectroscopic spectrum of normal light of the second embodiment.
Fig. 27 is a graph illustrating the spectroscopic spectrum of special light of the second embodiment.

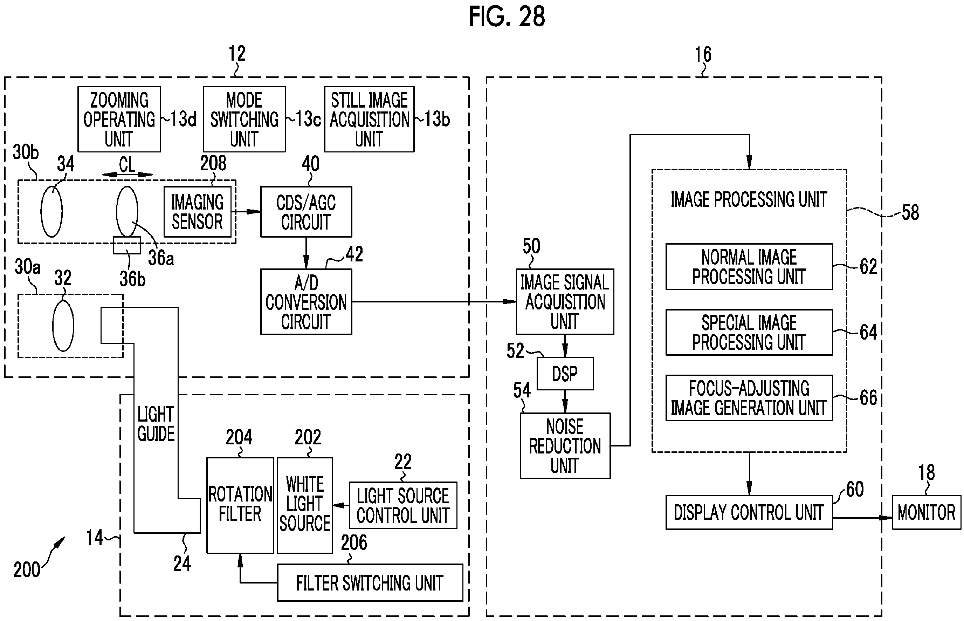

Fig. 28 is a block diagram illustrating the functions of an endoscope system of a third embodiment.
Fig. 29 is a plan view of a rotation filter.

### DESCRIPTION OF THE PREFERRED EMBODIMENTS

### [Embodiment 1A]

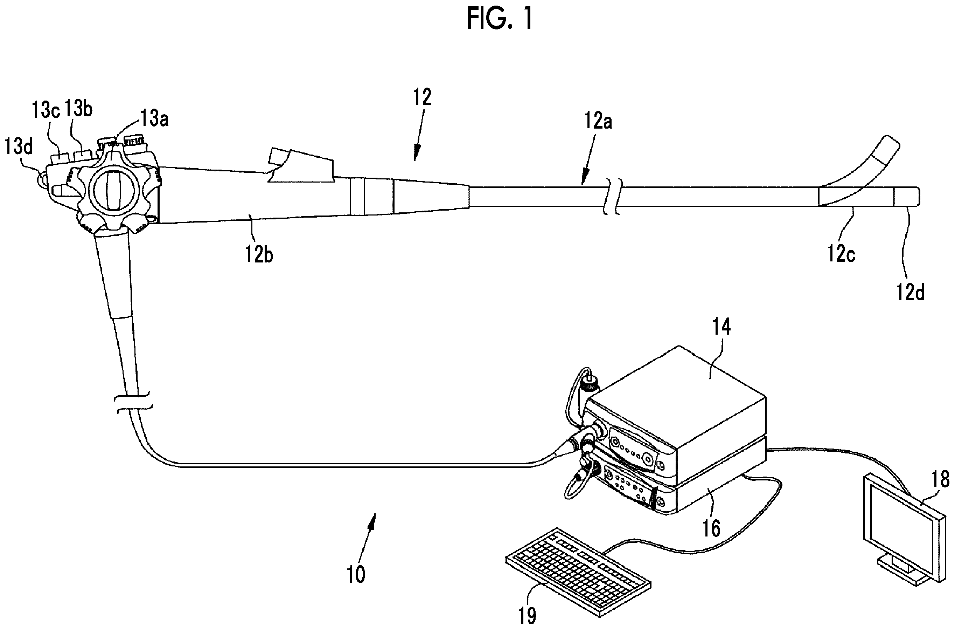

As illustrated in Fig. 1, an endoscope system 10 has an endoscope 12, a light source device 14, a processor device 16, a monitor 18 (display unit), and a console 19. The endoscope 12 is optically connected to the light source device 14, and is electrically connected to the processor device 16. The endoscope 12 has an insertion part 12a to be inserted into a subject, an operating part 12b provided at a proximal end portion of the insertion part 12a, and a bending part 12c and a distal end part 12d provided on a distal end side of the insertion part 12a. By operating an angle knob 13a of the operating part 12b, the bending part 12c makes a bending motion. The distal end part 12d is directed in a desired direction by this bending motion.

Additionally, the operating part 12b is provided with a still image acquisition unit 13b used for operating the acquisition of still images, a mode switching unit 13c used for operating the switching of observation modes, and a zooming operating unit 13d used for operating the change of a zoom magnification factor, in addition to the angle knob 13a. In the still image acquisition unit 13b, a freeze operation of displaying a still image of an observation target on the monitor 18, and a release operation of saving the still image in a storage are possible.

The endoscope system 10 has a normal mode, a special mode, and an individual-tissue display mode as the observation modes. In a case where an observation mode is the normal mode, normal light obtained by combining a plurality of colors of light components together in a quantity-of-light ratio Lc for normal mode is emitted, and a normal image is displayed on a monitor 18 on the basis of image signals obtained by imaging the observation target under illumination with this normal light. Additionally, in a case where an observation mode is the special mode, special light obtained by combining a plurality of colors of light components together in a quantity-of-light ratio Ls for special mode is emitted, and a special image is displayed on the monitor 18 on the basis of image signals obtained by imaging the observation target under illumination with this special light.

Additionally, in a case where an observation mode is the individual-tissue display mode, the special light is emitted, and an individual-tissue image for clearly distinguishing and displaying various tissues (refer to Figs. 11 and 13), such as a sub epithelial capillary, a marginal crypt epithelium, an intervening part, and a crypt opening, respectively, is displayed on the monitor 18 on the basis of image signals obtained by imaging the observation target under illumination with the special light. The sub epithelial capillary and the like displayed in the individual-tissue image becomes observable by operating the zooming operating unit 13d to magnifying the observation target.

The processor device 16 is electrically connected to the monitor 18 and the console 19. The monitor 18 outputs and displays an image of the observation target, information accompanying the image, and the like. The console 19 functions as a user interface that receives input operations, such as designation or the like of a region of interest (ROI) and function setting.

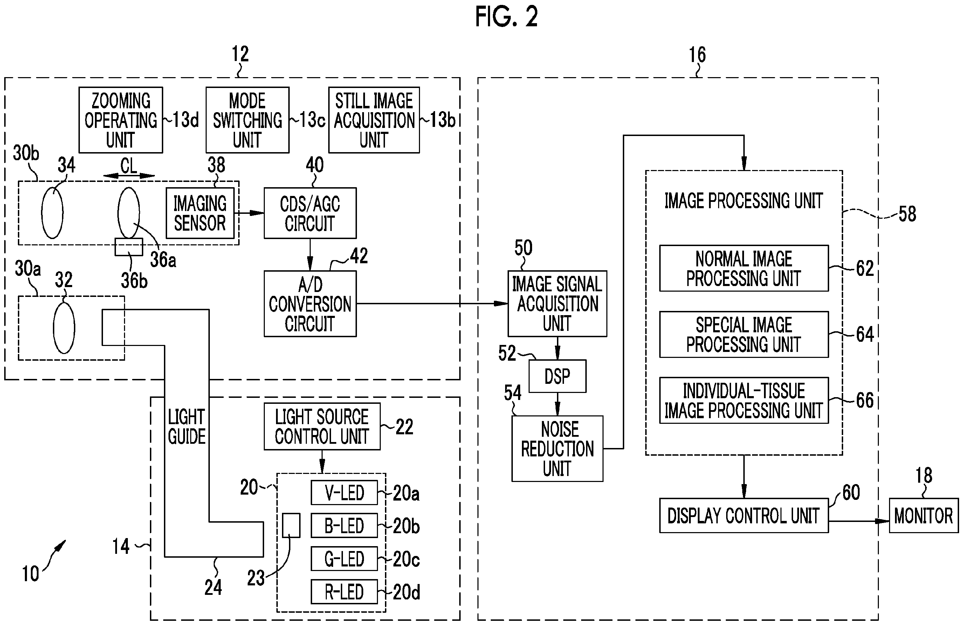

As illustrated in Fig. 2, the light source device 14 comprises a light source 20 that emits the illumination light to be used for illumination of the observation target, and a light source control unit 22 that controls the light source 20. The light source 20 is semiconductor light sources, such as a plurality of colors of light emitting diodes (LEDs). The light source control unit 22 controls the quantity of light emission of the illumination light by ON/OFF of the LEDs and the adjustment of the driving currents or driving voltages of the LEDs. Additionally, the light source control unit 22 controls the wavelength range of the illumination light, for example, by changing the optical filters.

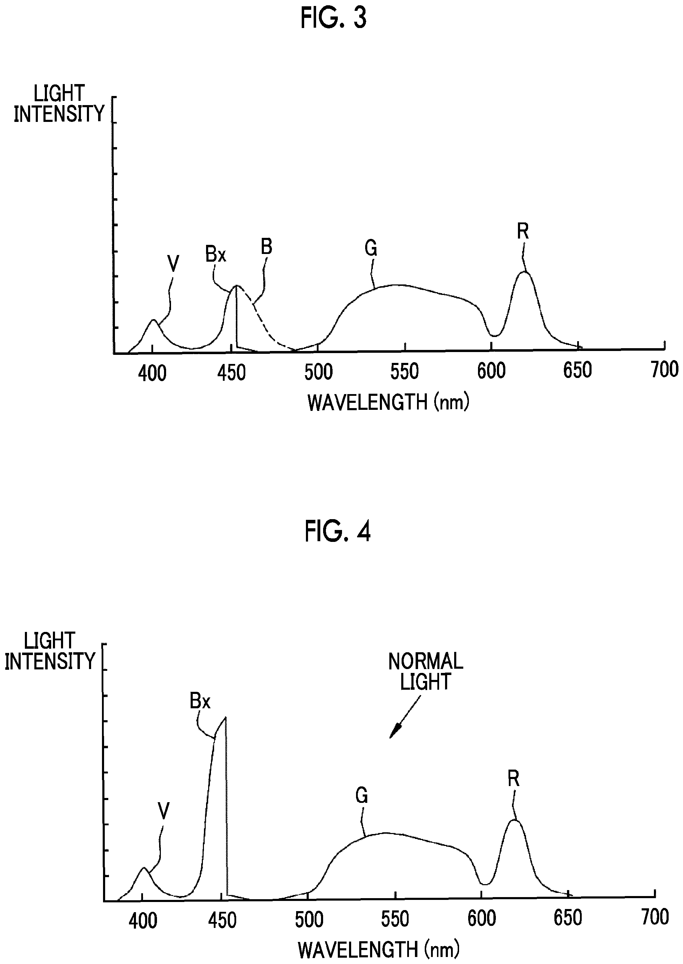

In the first embodiment, the light source 20 has four color LEDs of a violet light emitting diode (V-LED) 20a, a blue light emitting diode (B-LED) 20b, a green light emitting diode (G-LED) 20c, a red light emitting diode (R-LED) 20d, and a wavelength cutoff filter 23. As illustrated in Fig. 3, the V-LED 20a emits violet light V having a wavelength range of 380 nm to 420 nm.

The B-LED 20b emits blue light B having a wavelength range of 420 nm to 500 nm. The blue light B emitted from the B-LED 23b is cut by the wavelength cutoff filter 23 on at least a longer wavelength side than the peak wavelength 450 nm. Accordingly, the blue light Bx after being transmitted through the wavelength cutoff filter 23 has a wavelength range of 420 to 460 nm. In this way, the reason why light in a wavelength range on the longer wavelength side than 460 nm is cut is that the light in the wavelength range on the longer wavelength side than 460 nm is a factor in which the blood vessel contrast of blood vessels that is the observation target is lowered. In addition, the wavelength cutoff filter 23 may reduce the light in the wavelength range on the longer wavelength side than 460 nm instead of cutting the light in the wavelength range on the longer wavelength side than 460 nm.

The G-LED 20c emits green light G having a wavelength range of 480 nm to 600 nm. The R-LED 20d emits red light R having a wavelength range of 600 nm to 650 nm. In addition, center wavelengths and peak wavelengths of the respective colors of light emitted from the LEDs 20a to 20d may be the same as each other or may be different from each other.

The light source control unit 22 independently controls ON/OFF of the respective LEDs 20a to 20d, the quantity of light emission at the time of ON, and the like, thereby adjusting the light emission timing of illumination light, a light emission period, the quantity of light, and a spectroscopic spectrum. The control of ON and OFF in the light source control unit 22 varies in the respective observation modes.

In the case of the normal mode, the light source control unit 22 turns on the V-LED 20a, the B-LED 20b, the G-LED 20c, and the R-LED 20d altogether. In that case, as illustrated in Fig. 4, the quantity-of-light ratio Lc between the violet light V, the blue light B, the green light G, and the red light R is set such that the quantity of light emission of the blue light Bx becomes larger than the quantity of light emission of any of the violet light V, the green light G, and the red light R. Accordingly, in the normal mode, multicolor light for normal mode including the violet light V, the blue light Bx, the green light G, and the red light R is emitted as the normal light from the light source device 14. Since the normal light has an intensity equal to or more than a given level from a blue range to a red range, the normal light is substantially white.

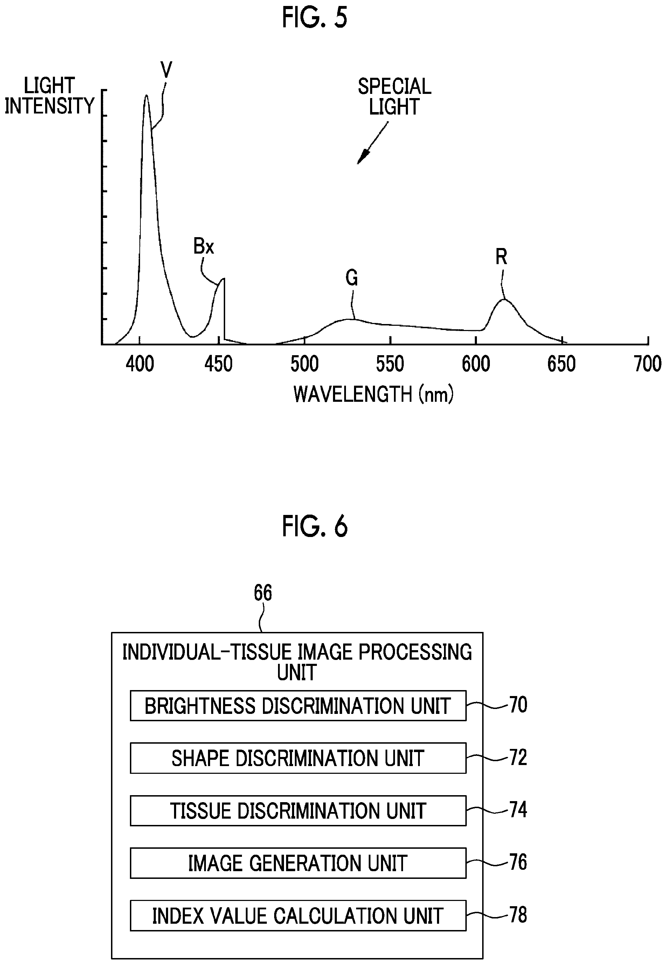

Even in the case of the special mode or the individual-tissue display mode, the light source control unit 22 turns on the V-LED 20a, the B-LED 20b, the G-LED 20c, and the R-LED 20d altogether. In that case, as illustrated in Fig. 5, the quantity-of-light ratio Ls between the violet light V, the blue light B, the green light G, and the red light R is set such that the quantity of light emission of the violet light V becomes larger than the quantity of light emission of any of the blue light Bx, the green light G, and the red light R and such that the green light G and the red light R become smaller than the violet light V and the blue light Bx. Accordingly, in the special mode or the individual-tissue display mode, multicolor light for special mode or individual-tissue display mode including the violet light V, the blue light Bx, the green light G, and the red light R is emitted as the special light from the light source device 14. Since the quantity of light emission of the violet light V is large, the special light is bluish light.

As illustrated in Fig. 2, the illumination light emitted from the light source 20 enters a light guide 24 inserted into the insertion part 12a via a light path coupling part (not illustrated) formed with a mirror, a lens, or the like. The light guide 24 is built in the endoscope 12 and a universal cord, and propagates the illumination light up to the distal end part 12d of the endoscope 12. The universal cord is a cord that connects the endoscope 12, and the light source device 14 and the processor device 16 together. In addition, multimode fiber can be used as the light guide 24. As an example, a fine-diameter fiber cable of which the core diameter is 105 µm, the clad diameter is 125 µm, and a diameter including a protective layer used as an outer cover is µ0.3 mm to 0.5 mm can be used for the light guide 24.

The distal end part 12d of the endoscope 12 is provided with an illumination optical system 30a and an imaging optical system 30b. The illumination optical system 30a has an illumination lens 32. The observation target is illuminated with the illumination light propagated through the light guide 24 via the illumination lens 32. The imaging optical system 30b has an objective lens 34, a magnifying optical system 36, and an imaging sensor 38. Various kinds of light, such as reflected light from the observation target, scattered light, and fluorescence, enters the imaging sensor 38 via the objective lens 34 and the magnifying optical system 36. Accordingly, the image of the observation target is formed on the imaging sensor 38.

The magnifying optical system 36 comprises a zoom lens 36a that magnifies the observation target, and a lens drive unit 36b that moves the zoom lens 36a in an optical axis direction CL. The zoom lens 36a magnifies or reduces the observation target of which the image is formed on the imaging sensor 38 by freely moving between a telephoto end and a wide end in accordance with a zoom control performed by the lens drive unit 36b.

The imaging sensor 38 is a color imaging sensor that images the observation target irradiated with the illumination light. Each pixel of the imaging sensor 38 is provided with any one of a red (R) color filter, a green (G) color filter, and a blue (B) color filter. The imaging sensor 38 receives blue light with the B pixel provided with the B color filter from violet, receives green light with a G pixel provided with the G color filter, and receives red light with an R pixel provided with the R color filter. Image signals of respective RGB colors are output from the respective color pixels. The imaging sensor 38 transmits the output image signals to a CDS/AGC circuit 40.

In the normal mode, the imaging sensor 38 images the observation target illuminated with the normal light, thereby outputting a Bc image signal from the B pixel, outputting a Gc image signal from the G pixel, and outputting an Rc image signal from the R pixel. Additionally, in the special mode or the individual-tissue display mode, the imaging sensor 38 images the observation target illuminated with the special light, thereby outputting a Bs image signal from the B pixel, outputting a Gs image signal from the G pixel, and outputting an Rs image signal from the R pixel.

As the imaging sensor 38, a charge coupled device (CCD) imaging sensor, a complementary metal-oxide semiconductor (CMOS) imaging sensor, or the like is available. Additionally, instead of the imaging sensor 38 provided with the color filters in the primary colors of RGB, a complementary color imaging sensor comprising complementary color filters in C (cyan), M (magenta), Y (yellow), and G (green) may be used. In a case where the complementary color imaging sensor is used, image signals of four colors of CMYG are output. For this reason, the same respective RGB image signals as those in the imaging sensor 38 can be obtained by converting the image signals of four colors of CMYG into image signals of three colors of RGB through color conversion between complementary colors and the primary colors. Additionally, instead of the imaging sensor 38, a monochrome sensor that is not provided with the color filters may be used.

The CDS/AGC circuit 40 performs correlated double sampling (CDS) and automatic gain control (AGC) on analog image signals received from the imaging sensor 38. An analog-to-digital (A/D) conversion circuit 42 converts the analog image signals, which have passed through the CDS/AGC circuit 40, into digital image signals. The A/D conversion circuit 42 inputs the digital image signals after the A/D conversion to the processor device 16.

The processor device 16 comprises an image signal acquisition unit 50, a digital signal processor (DSP) 52, a noise reduction unit 54, an image processing unit 58, and a display control unit 60. Hardware structures of the respective units within the processor device are various processors as shown below. Various processors include exclusive electric circuits, which are processors having circuit configurations exclusively designed to execute specific processing (specific calculation), such as a central processing unit (CPU) that is a general-purpose processor that executes software (programs) to function as various processing units, a programmable logic device (PLD) that is a processor capable of changing a circuit configuration after manufacture of a field programmable gate array (FPGA) or the like, and an application specific integrated circuit (ASIC). In addition, the same applies the respective units inside the endoscope 12 and the light source device 14.

The image signal acquisition unit 50 (equivalent to the "image acquisition unit" of the invention) acquires digital image signals corresponding to the observation modes from the endoscope 12. In the case of the normal mode, the Bc image signal, the Gc image signal, and the Rc image signal are acquired. In the case of the special mode or the individual-tissue display mode, the Bs image signal, the Gs image signal, and the Rs image signal are acquired. By acquiring these image signals, the image signal acquisition unit 50 acquires images in a plurality of bands.

The DSP 52 performs various kinds of signal processing, such as defect correction processing, offset processing, gain correction processing, linear matrix processing, gamma conversion processing, demosaicing processing, and the like, on the image signals acquired by the image signal acquisition unit 50. In the defect correction processing, a signal of a defective pixel of the imaging sensor 38 is corrected. In the offset processing, a dark current component is removed from the image signals subjected to the defect correction processing, and an accurate zero level is set. In the gain correction processing, a signal level is adjusted by multiplying the image signals subjected to the offset processing by a specific gain.

The linear matrix processing enhances color reproducibility on the image signals subjected to the gain correction processing. In the gamma conversion processing, brightness and saturation of image signals subjected to the linear matrix processing are adjusted. By performing the demosaicing processing (also referred to as equalization processing or synchronization processing) on the image signals subjected to the gamma conversion processing, a signal of a color that runs short in each pixel is generated by interpolation. By means of this demosaicing processing, all pixels have signals of respective RGB colors. The noise reduction unit 54 performs noise reducing processing using, for example, a moving average method, a median filter method, or the like on the image signals subjected to the demosaicing processing or the like by the DSP 52, and reduces noise.

The image processing unit 58 (specific calculation processing unit) comprises a normal image generation unit 62, a special image processing unit 64, and an individual-tissue image processing unit 66. The normal image processing unit 62 operates in a case where the normal mode is set, and performs color conversion processing, color enhancement processing, and structure enhancement processing on the received Bc image signal, Gc image signal, and Rc image signal. In the color conversion processing, color conversion processing is performed on the RGB image signals by 3x3 matrix processing, gradation transformation processing, three-dimensional look-up table (LUT) processing, and the like.

The color enhancement processing is performed on the RGB image signals subjected to the color conversion processing. The structure enhancement processing is the processing of enhancing the structure of the observation target, and is performed on the RGB image signals after the color enhancement processing. The normal image is obtained by performing the various kinds of image processing as described above. Since the normal image is an image obtained on the basis of the normal light in which the violet light V, the blue light Bx, the green light G, and the red light R are emitted in a well-balanced manner, the normal image is a natural-tone image. The normal image is input to the display control unit 60.

The special image processing unit 64 operates in a case where the special mode is set. In the special image processing unit 64, the color conversion processing, the color enhancement processing, and the structure enhancement processing is performed on the received Bs image signal, Gs image signal, and Rs image signal. The processing contents of the color conversion processing, the color enhancement processing, and the structure enhancement processing are the same as those of the normal image processing unit 62. The special image is obtained by performing the various kinds of image processing as described above. Since the special image is an image obtained on the basis of special light in which the violet light V with a high absorption coefficient of hemoglobin of blood vessels has a larger quantity of light emission than the blue light Bx, the green light G, and the red light R in the other colors, the resolution of a blood vessel structure or a glandular structure is higher than that of the other structures. The special image is input to the display control unit 60.

The individual-tissue image processing unit 66 operates in a case where the individual-tissue display mode is set. In the individual-tissue image processing unit 66, the individual-tissue image is generated by performing the processing, for clearly distinguishing and displaying the various tissues, on the received Bs image signal, Gs image signal, and Rs image signal.. Additionally, index values obtained by indexing the various tissues are calculated on the basis of the generated individual-tissue image. The calculation of the individual-tissue image and the generation of the index values will be described below in detail. The individual-tissue image or the like is input to the display control unit 60. The processing performed by image processing unit 58 refers to "specific calculation processing" and the image (normal image, special image, individual-tissue image, or the like) obtained by performing the specific calculation processing refers to "calculated image".

The display control unit 60 performs a display control for displaying an image on the monitor 18 from the image processing unit 58. In a case where the normal mode, the display control unit 60 performs the control of displaying the normal image on the monitor 18. In a case where the special mode is set, the display control unit 60 performs the control of displaying the special image on the monitor 18. In a case where the individual-tissue display mode, the display control unit 60 performs the control of displaying the individual-tissue image on the monitor 18.

As illustrated in Fig. 6, the individual-tissue image processing unit 66 comprises a brightness discrimination unit 70, a shape discrimination unit 72, a tissue discrimination unit 74, and an image generation unit 76 in order to perform the processing of clearly distinguishing and displaying various tissues (refer to Figs. 11 and 13), such as the sub epithelial capillary, the marginal crypt epithelium, the intervening part, and the crypt opening, respectively. Additionally, the individual-tissue image processing unit 66 comprises an index value calculation unit 78 in order to calculate the index values obtained by indexing the various tissues.

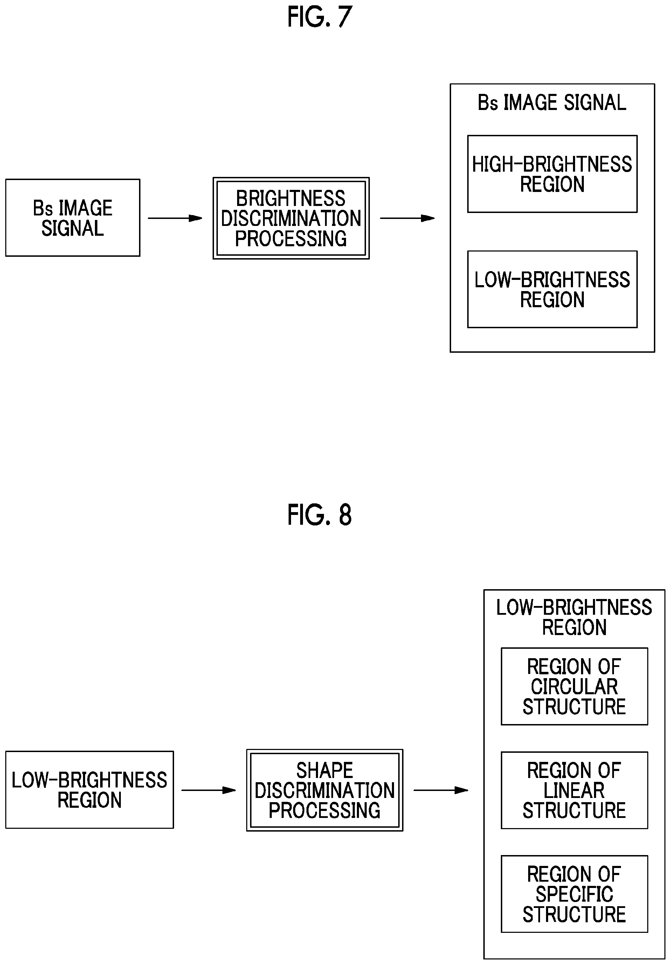

In the brightness discrimination unit 70, the brightness discrimination processing of discriminating whether or not a brightness value is equal to more than a specific threshold value Th is performed on the Bs image signal among the received image signals. In the brightness discrimination processing, as illustrated in Fig. 7, in the Bs image signal, a region where the brightness value is equal to or more than the specific threshold value Th is discriminated as a high-brightness region, and a region where the brightness value is less than the specific threshold value Th is discriminated as a low-brightness region. In addition, although the brightness discrimination processing is performed on the Bs image signal in which the resolution of the various tissues, such as the sub epithelial capillary and the marginal crypt epithelium, is high. However, the brightness discrimination processing may be performed to other image signals, such as the Gs image signal as long as the resolution of the various tissues is high.

The shape discrimination unit 72 performs the shape discrimination processing of discriminating the shape of a structure, which is included in the low-brightness region, in the Bs image signal. In the shape discrimination processing, as illustrated in Fig. 8, three pattern regions of a region of a circular structure, a region of a linear structure, and a region of a specific structure that does not belong to either the circular structure or the linear structure is detected from the low-brightness region. In addition, as the shape discrimination processing, for example, it is preferable to use pattern matching processing, morphology processing, Hessian analysis, or the like.

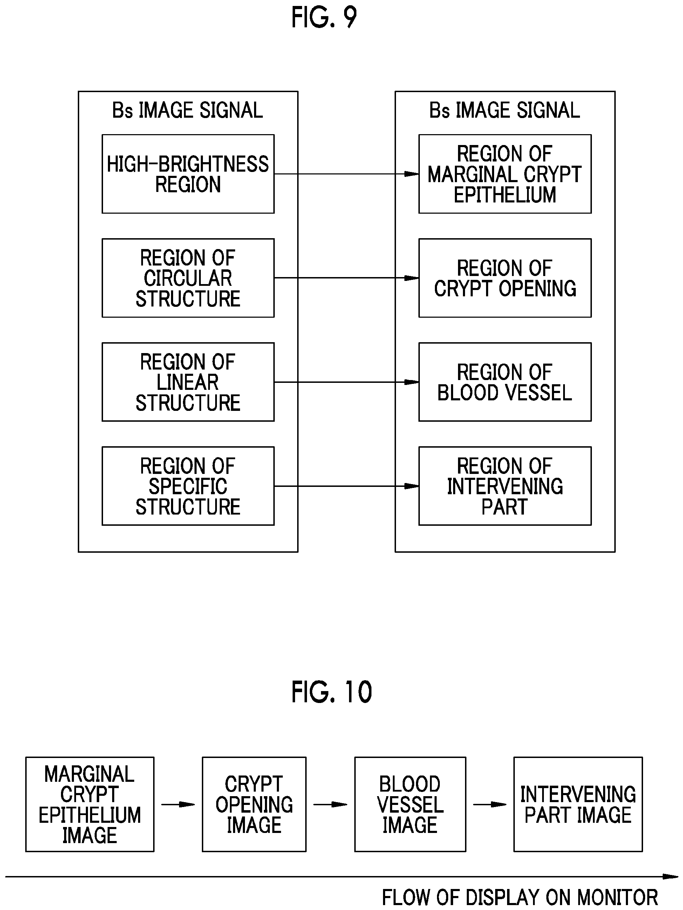

The tissue discrimination unit 74 performs discrimination of tissue among candidates of the tissue included in the high-brightness region and the low-brightness region on the basis of the Bs image signal subjected to the shape discrimination processing and the brightness discrimination processing, and discriminates tissue in accordance with shape discrimination results with respect to candidates of the tissue included in the low-brightness region. Specifically, the tissue discrimination unit 74 performs the tissue discrimination processing of discriminating the regions of the four tissues including the sub epithelial capillary, the marginal crypt epithelium, the intervening part, and the crypt opening. As illustrated in Fig. 9, in the Bs image signal, the high-brightness region is discriminated as being a region of the marginal crypt epithelium. Additionally, in the Bs image signal, the region of the circular structure is discriminated as being a region of the crypt opening. Additionally, in the Bs image signal, the region of the linear structure is discriminated as a region of the blood vessel (sub epithelial capillary). Additionally, in the Bs image signal, the region of the specific structure is discriminated as being a region of the intervening part. In addition, in the tissue discrimination unit 74, the discrimination of the tissue is performed on the candidates of the tissue included in the low-brightness region in accordance to the shape discrimination results. Instead of or in addition to this, the discrimination of the tissue may be performed on the candidates of the tissue included in the high-brightness region in accordance with the shape discrimination results.

The image generation unit 76 (corresponding to "an individual-tissue image generation unit" of the invention) generates the individual-tissue image in which the various tissues are clearly distinguished from each other, on the basis of the Bs image signal, the Gs image signal, and the Rs image signal that have been subjected to the tissue discrimination processing. In the individual-tissue image, the various tissues may be displayed in mutually different colors, respectively. Additionally, in the individual-tissue image, the tissue set in advance among the various tissues may be displayed in a further enhanced manner than the other tissue. Additionally, as illustrated in Fig. 10, a marginal crypt epithelium image obtained by extracting the region of the marginal crypt epithelium from the Bs image signal subjected to the tissue discrimination, a crypt opening image obtained by extracting the region of the crypt opening from the Bs image signal subjected to the tissue discrimination, a blood vessel image obtained by extracting the region of the blood vessel (sub epithelial capillary) from the Bs image signal subjected to the tissue discrimination, and an intervening part image obtained by extracting the intervening part from the Bs image signal subjected to the tissue discrimination may be displayed as the individual-tissue image in accordance with a preset specific display timing.

A method of discriminating the above tissue and a method of generating the individual-tissue image will be described taking a case where the tissue of a stomach is normal, and a case where the structure of the stomach is irregular as examples. For example, in the tissue of the stomach in a normal state, as illustrated in Fig. 11, pits in the tissue extend in a depth direction, and the respective pits are regularly aligned at regular intervals. Therefore, the structure appearing on a tissue surface is also a structure with given regularity. For example, the sub epithelial capillary (SEC) has a polygonal low-brightness linear structure. The marginal crypt epithelium (MCE) is arranged inside the sub epithelial capillary, and has a high-brightness structure. Additionally, the crypt opening (CO) has a low-brightness circular structure. Additionally, the intervening part (IP) has a low-brightness structure, and a distance between intervening parts is substantially constant.

By performing the brightness discrimination processing, the shape discrimination processing, and the tissue discrimination processing on the image obtained by imaging the tissue illustrated in Fig. 11, the image can be reliably sorted into four regions of the region of the sub epithelial capillary SEC, the region of the marginal crypt epithelium MCE, the region of the crypt opening CO, and the region of the intervening part IP. Additionally, in a case where setting of displaying the region of the marginal crypt epithelium MCE in an enhanced manner is performed in the image generation unit 76, as illustrated in Fig. 12, an individual-tissue image Px in which the region of the marginal crypt epithelium MCE (corresponding to "specific tissue" of the invention) is enhanced more than the other regions is displayed on the monitor 18.

Additionally, in the tissue of the stomach in a state in which the structure is irregular, as illustrated in Fig. 13, a direction in which the pits in the tissue extend vary depending on pits, and the arrangement intervals of the respective pits also vary. For that reason, the regularity of the structure appearing on the tissue surface collapses in the normal case. For example, the sub epithelial capillary SEC has a low-brightness linear structure that is not closed because the shape of the polygonal linear structure collapses in the normal case. The marginal crypt epithelium MCE varies greatly in structure as compared to the normal case, and has a polygonal high-brightness structure. Additionally, the crypt opening CO disappears unlike the normal case. Additionally, the intervening part (IP) varies in length for each intervening part due to irregular pits.

By performing the brightness discrimination processing, the shape discrimination processing, and the tissue discrimination processing on the image obtained by imaging the tissue illustrated in Fig. 13, the image can be reliably sorted into three regions of the region of the sub epithelial capillary SEC, the region of the marginal crypt epithelium MCE, and the region of the intervening part IP. Additionally, in a case where setting of displaying the region of the marginal crypt epithelium MCE in an enhanced manner is performed in the image generation unit 76, as illustrated in Fig. 14, an individual-tissue image Py in which the region of the marginal crypt epithelium MCE (corresponding to "specific tissue" of the invention) is enhanced more than the other regions is displayed on the monitor 18.

The index value calculation unit 78 calculates a marginal crypt epithelium index value by extracting the region of the marginal crypt epithelium from the Bs image signal subjected to the tissue discrimination to index the marginal crypt epithelium. The marginal crypt epithelium index value includes, for example, the density or the like of the marginal crypt epithelium in a given range. Similarly, the index value calculation unit 78 calculates a crypt opening index value by extracting the region of the crypt opening from the Bs image signal subjected to the tissue discrimination to index the crypt opening. The index value calculation unit 78 calculates a blood vessel index value by extracting the region of the blood vessel (sub epithelial capillary) from the Bs image signal subjected to the tissue discrimination to index the blood vessel. The index value calculation unit 78 calculates an intervening part index value by extracting the region of the intervening part from the Bs image signal subjected to the tissue discrimination to index the intervening part. In addition, as a method for the indexing, for example, there is a method of calculating the density in the entire image or a given image region. Additionally, an index value image displayed in different colors in accordance with the index values calculated in the index value calculation unit 78 may be generated and displayed on the monitor 18.

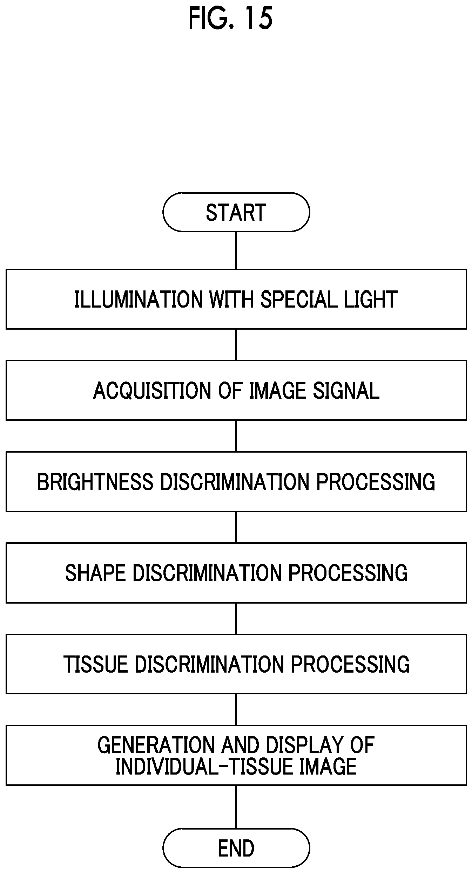

Next, a series of flow of the individual-tissue display mode will be described using a flowchart illustrated in Fig. 15. In a case where the individual-tissue display mode is set by the mode switching unit 13c, the observation target is irradiated with the special light. The imaging sensor 38 outputs the Bs image signal, the Gs image signal, and the Rs image signal by imaging the observation target illuminated with the special light.

Next, the brightness discrimination processing is performed on the Bs image signal. As a result of the brightness discrimination processing, the Bs image signal is divided into two regions of the high-brightness region and the low-brightness region. The shape discrimination processing is performed on the low-brightness region of the Bs image signal. As a result of the shape discrimination processing, the low-brightness region is divided into three regions of the region of the circular structure, the region of the linear structure, and the region of the specific structure. The tissue discrimination processing of discriminating the various tissues is performed on the basis of the Bs image signal shape subjected to the brightness discrimination processing and the shape discrimination processing.

The high-brightness region of the Bs image signal is discriminated as the region of the marginal crypt epithelium as a result of the tissue discrimination processing. Additionally, the region of the circular structure of the Bs image signal is discriminated as the region of the crypt opening. Additionally, the region of the linear structure of the Bs image signal is discriminated as the region of the blood vessel (sub epithelial capillary). Additionally, the specific structure region of the Bs image signal is discriminated as the region of the intervening part. Then, the individual-tissue image in which the various tissues are clearly distinguished from each other is generated on the basis of the Bs image signal, the Gs image signal, and the Rs image signal that have been subjected to the tissue discrimination processing, and displayed on the monitor 18.

### [Embodiment 1B]

In addition, in the above Embodiment 1A, the discrimination between the blood vessel (sub epithelial capillary) and the intervening part is performed by the discrimination based on the shape. However, in a case where the resolution of the linear structure in the image is low, the discrimination of the blood vessel is difficult. Therefore, in such a case, the discrimination may be performed by methods other than the shape. Although the low brightness is common to the blood vessel and the intervening part, there is a difference in light absorption characteristics with respect to short-wavelength light. The blood vessel has a peak of absorption with respect to light near 420 nm, while the stroma included in the intervening part has a relatively flat absorption feature in a range of 410 to 460 nm. In Embodiment 1B, the discrimination between the blood vessel and the intervening part is performed on the basis of a difference in absorption with respect to such short-wave light.

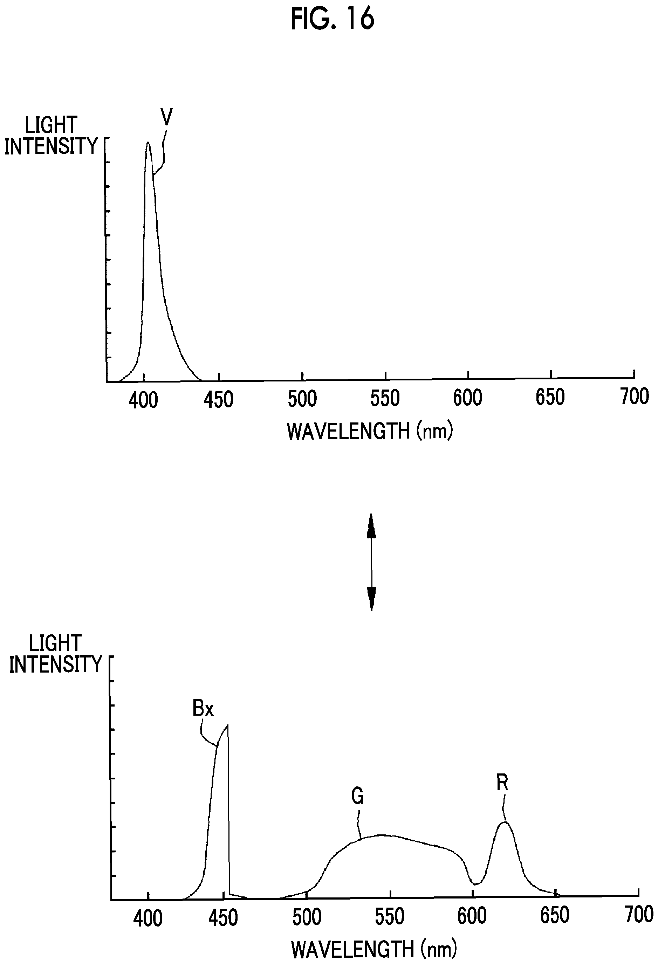

In Embodiment 1B, in order to obtain information on the light absorption of the blood vessel, as illustrated in Fig. 16, in a first frame, the observation target is irradiated with the violet light V and the observation target is imaged by the imaging sensor 38. Accordingly, a B1s image signal is output from a B pixel, a G1s image signal is output from the G pixel, and an R1s image signal is output from the R pixel. On the other hand, in order to obtain information on the light absorption of the stroma included in the intervening part, after the violet light is radiated, in the next second frame, the violet light V, the blue light Bx, the green light G, and the red light R are turned on and are illuminated toward the observation target, and this observation target is imaged by the imaging sensor 38. Accordingly, a B2s image signal is output from a B pixel, a G2s image signal is output from the G pixel, and an R2s image signal is output from the R pixel. In Embodiment 1B, the illumination with the violet light V and the illumination with the blue light Bx, the green light G, and the red light R are alternately performed.

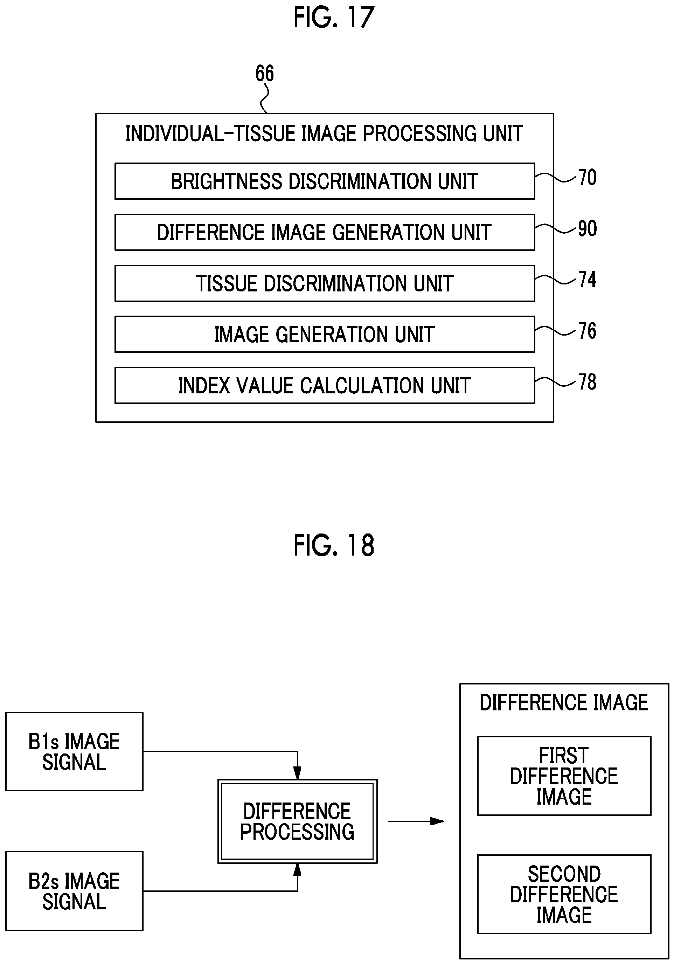

Additionally, in the individual-tissue image processing unit 66 of Embodiment 1B, as illustrated in Fig. 17, a difference image generation unit 90 is provided instead of the shape discrimination unit 72. In Embodiment 1B, the brightness discrimination unit 70 performs the discrimination between the high-brightness region and the low-brightness region on the basis of the B1s image signal or the B2s image signal. The difference image generation unit 90 performs difference processing of the B1s image signal and the B2s image signal to generate a difference image. Additionally, as illustrated in Fig. 18, the difference image generation unit 90 divides the generated difference image into two regions of a first difference region where a difference in signal value between the B1s image signal and the B2s image signal is equal to or more than a given value, and a second difference region where the difference in signal value between the B1s image signal and the B2s image signal is less than the given value.

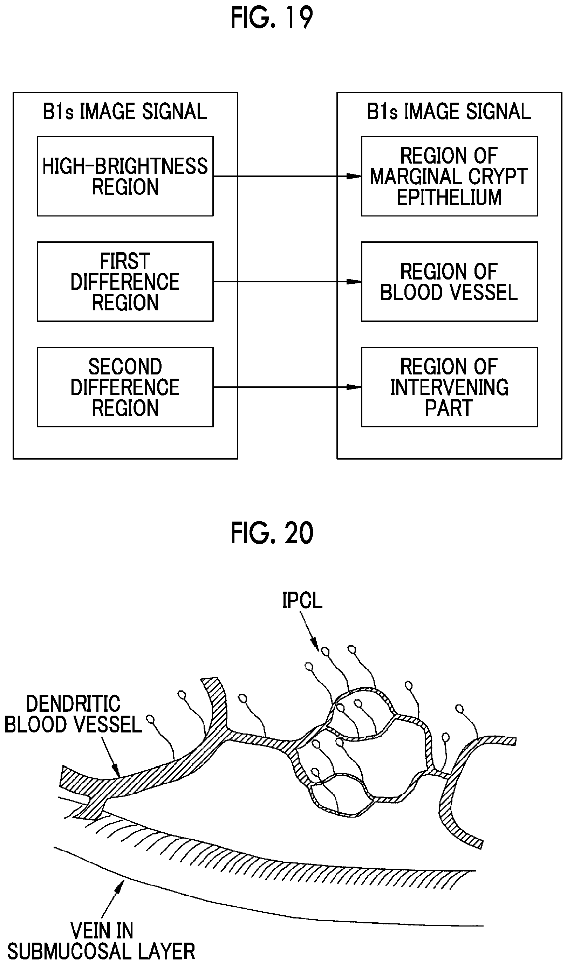

In the tissue discrimination unit 74, as illustrated in Fig. 19, the high-brightness region is discriminated as the region of the marginal crypt epithelium similarly to the above Embodiment 1A. On the other hand, the tissue discrimination unit 74 discriminates the region of the low-brightness region, which is discriminated as the first difference region in the difference image, as the region of the blood vessel. Additionally, the tissue discrimination unit 74 discriminates the region of the low-brightness region, which is discriminated as the second difference region in the difference image, as the region of the intervening part. In addition, a ratio image may be created instead of the difference image by performing ratio calculation processing of calculating the ratio of the B1s image signal and the B2s image signal. In this case, in the ratio image, a region where the ratio is out of a given range is discriminated as the region of the blood vessel, and a region where the ratio is within the given range is discriminated as the region of the intervening part.

### [Embodiment 1C]

In the above Embodiments 1A and 1B, although the discrimination of the tissue in the stomach, such as the sub epithelial capillary, the marginal crypt epithelium, the intervening part, and the crypt opening, will be described, the discrimination of the tissue of the invention is also possible also for regions other than the stomach. In Embodiment 1C, the tissue appearing in the esophagus is discriminated. In the esophagus, as illustrated in Fig. 20, the esophagus it does not have the glandular structure as tissue unlike the stomach, and has mainly two kinds of blood vessels of intra-epithelial papillary capillary loops (IPCL) and a dendritic blood vessel. These two kinds of blood vessels are discriminated in Embodiment 1C. In addition, in the esophagus, a vein in a submucosal layer is distributed at a position still deeper than the dendritic blood vessel.

In Embodiment 1C, a method of performing the brightness discrimination processing and the shape discrimination processing based on the Bs image signal among the image signals obtained by imaging the esophagus under illumination with the special light to perform the tissue discrimination is considered. The brightness discrimination processing is the same as that of Embodiment 1A. However, since the esophagus does not have the glandular structure that becomes the high-brightness region, only the low-brightness region is present in the Bs image signal subjected to the brightness discrimination processing. Next, shape discrimination processing is performed on the Bs image signal subjected to the brightness discrimination processing. As the shape discrimination processing, in Embodiment 1C, loop structure detection processing of detecting in a loop structure is performed.

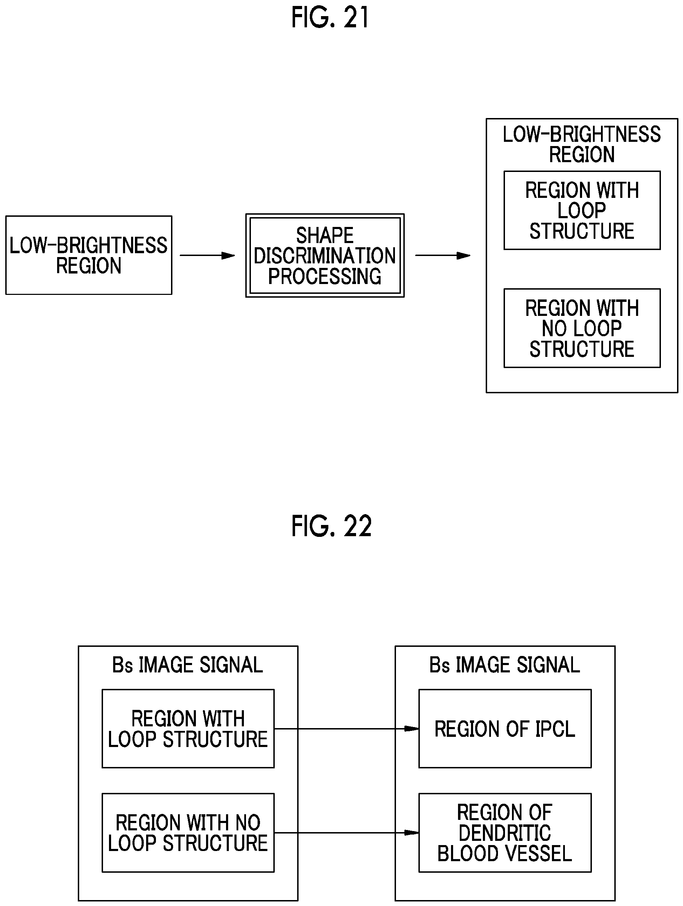

In the loop structure detection processing, after the linear structure shown in Embodiment 1A is detected, the curvature of the detected linear structure is analyzed and the presence or absence of the loop structure is detected. As illustrated in Fig. 21, the low-brightness region of the Bs image signal subjected to the above shape discrimination processing is divided into two regions of a region with the loop structure, and a region with no loop structure. The tissue discrimination processing is performed on the Bs image signal shape subjected to the shape discrimination processing. As a result, as illustrated in Fig. 22, the region with the loop structure is discriminated as a region of the IPCL, and the region with no loop structure is discriminated as a region of the dendritic blood vessel.

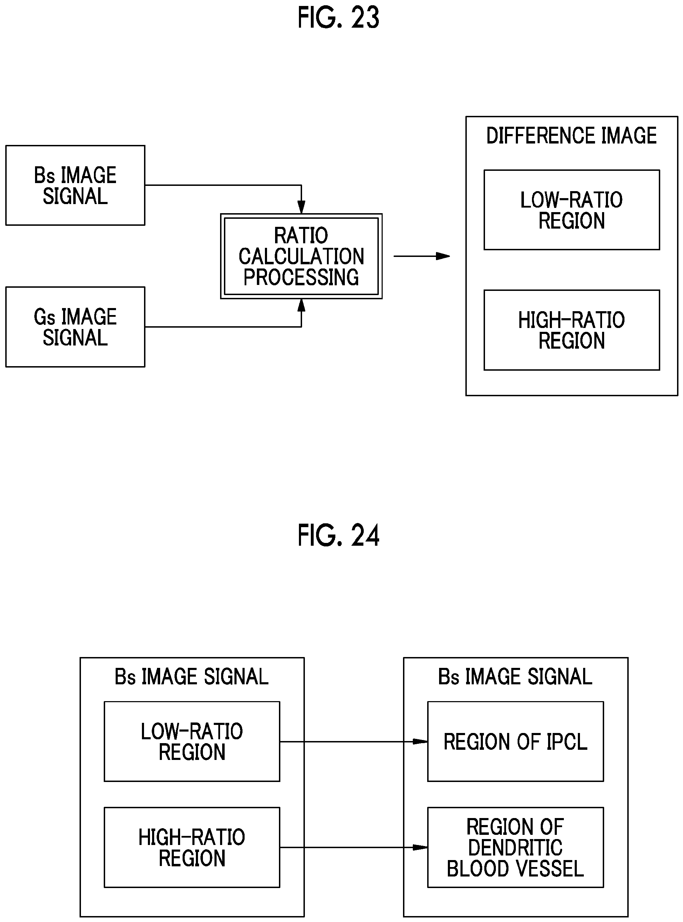

Additionally, in Embodiment 1C, a method of performing the brightness discrimination processing and the ratio calculation processing based on the Bs image signal and the Gs image signal among the image signals obtained by imaging the esophagus under illumination with the special light to perform the tissue discrimination is also considered. The brightness discrimination processing is the same above and, and Only the low-brightness region is included in the Bs image signal subjected to the brightness discrimination processing. On the other hand, in the ratio calculation processing, as illustrated in Fig. 23, a ratio Bs/Gs of the Bs image signal and the Gs image signal is calculated. Then, in the ratio calculation processing, the calculated ratio Bs/Gs is divided into two regions of a low-ratio region equal to or less than a given value, and a high-ratio region where the ratio Bs/Gs exceeds the given value. In the tissue discrimination processing, as illustrated in Fig. 24, the low-ratio region is discriminated as the region of the IPCL, and the high-ratio region is discriminated as the region of the dendritic blood vessel.

As described above, due to the ratio Bs/Gs, the IPCL and the dendritic blood vessel can be discriminated from each other on the basis of the following reasons. Since the Bs image signal is an image obtained on the basis of short-wave light with low deepness, such as the violet light V, the visibility of the IPCL distributed at a shallow position of tissue is high in the Bs image signal, but the visibility of the dendritic blood vessel at a deep position of the tissue is low. In contrast, since the Gs image signal is an image obtained on the basis of long-wave light with high deepness, such as the green light G, in the Gs image signal, the dendritic blood vessel has high contrast and high visibility. Meanwhile, in the long-wave light, such as the green light G, the absorption feature of blood is low. In the Gs image signal, thin blood vessels, such as IPCL, have low visibility. The followings can be said from the above consideration.
- The signal value of the Bs image signal is low, and the signal value of the Gs image signal is high → Bs/Gs is small → IPCL
- The signal value of the Bs image signal is high, and the signal value of the Gs image signal is low → Bs/Gs is large → Dendritic blood vessel

### [Second Embodiment]

In a second embodiment, the observation target is illuminated using a laser light source and a fluorescent body instead of the four-color LEDs 20a to 20d illustrated in the above Embodiments 1A and 1B. In the following, only portions different from Embodiments 1A and 1B will be described, and description of substantially the same portions as those of the first embodiment will be omitted.

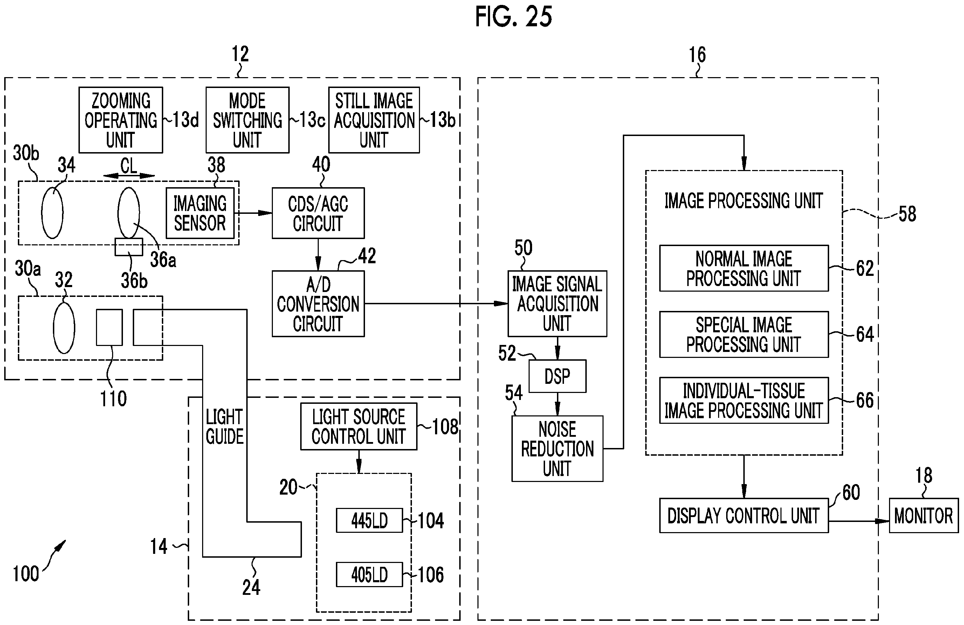

As illustrated in Fig. 25, in the endoscope system 100 of the second embodiment, in the light source 20 of the light source device 14, a blue laser light source that emits blue laser light having a central wavelength of 445 ± 10 nm (written as "445LD"; LD represents Laser Diode) 104 and a blue-violet laser light source (written as "405LD") 106 that emits blue-violet laser light having a central wavelength of 405 ± 10 nm are provided instead of the four-color LEDs 20a to 20d. The light emission from semiconductor light-emitting elements of the respective light sources 104 and 106 are individually controlled by a light source control unit 108, and the quantity-of-light ratio of the emitted light of the blue laser light source 104 and the emitted light of the blue-violet laser light source 106 is changeable.

The light source control unit 108 turns on the blue laser light source 104 in the case of the normal mode. In contrast, in the case of the special mode or the individual-tissue display mode, both the blue laser light source 104 and the blue-violet laser light source 106 are turned on, and the light emission ratio of the blue laser light is controlled to become larger than the light emission ratio of the blue-violet laser light.

In addition, it is preferable that the half-width of the blue laser light or the blue-violet laser light is about ±10 nm. Additionally, as the blue laser light source 104 and the blue-violet laser light source 106, broad area type InGaN-based laser diodes can be utilized, and InGaNAs-based laser diodes and GaNAsb-based laser diodes can also be used. Additionally a configuration using a light emitter, such as a light emitting diode, may be adopted as the above light source.

The illumination optical system 30a is provided with a fluorescent body 110 that the blue laser light or the blue-violet laser light from the light guide 24 enters in addition to the illumination lens 32. The fluorescent body 110 is excited by the blue laser light to emit fluorescence. Additionally, a portion of the blue laser light is transmitted through the fluorescent body 110 without exciting the fluorescent body 1 10. The blue-violet laser light is transmitted through the fluorescent body 110 without exciting the fluorescent body 110. The inside of the body of the observation target is illuminated with the light emitted from the fluorescent body 110 via the illumination lens 32.

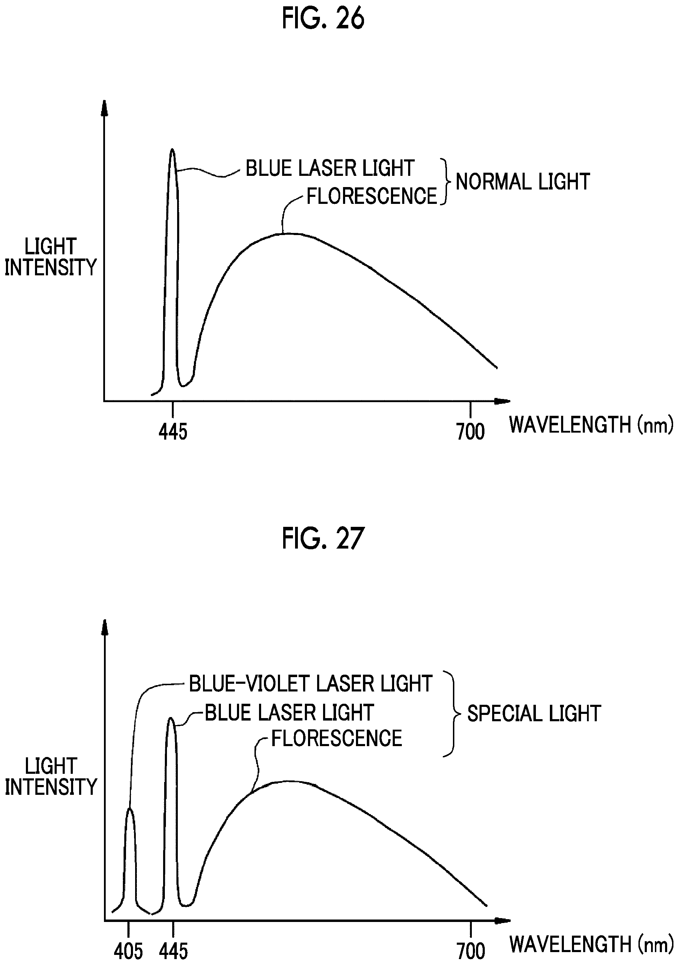

Here, in the normal mode, mainly, the blue laser light enters the fluorescent body 110. Therefore, the broadband light for normal mode, which is obtained by combining the blue laser light (with the fluorescence excited and emitted from the fluorescent body 110 due to the blue laser light as illustrated in Fig. 26, is illuminated to the observation target as the normal light. By imaging the observation target illuminated with the normal light by the imaging sensor 38, the normal image including the Bc image signal, the Gc image signal, and the Rc image signal is obtained.

On the other hand, in the special mode or the individual-tissue display mode, the blue-violet laser light and the blue laser light enter the fluorescent body 110. Therefore, the broadband light for special mode or the individual-tissue display mode, which is obtained by combining the blue-violet laser light, the blue laser light, and the fluorescence excited and emitted from the fluorescent body 110 due to the blue laser light together as illustrated in Fig. 27, is illuminated to the observation target as the special light. By imaging the observation target illuminated with the special light by the imaging sensor 38, the special image including the Bs image signal, the Gs image signal, and the Rs image signal is obtained.

In addition, as the fluorescent body 110, it is preferable to use those configured to include a plurality of types of fluorescent bodies (for example, a YAG-based fluorescent body or fluorescent bodies, such as BAM (BaMgAl₁₀O₁₇)) that absorb a portion of the blue laser light and are excited to emit light in green to yellow. As in the present configuration example, in a case where the semiconductor light-emitting elements are used as the excitation light sources of the fluorescent body 110, high-sensitive white light with a high emission ratio can be acquired, the intensity of the white light can be easily adjusted, and changes in color temperature and chromaticity of the white light can be suppressed to be small.

### [Third Embodiment]

In the third embodiment, the observation target is illuminated using a white light source, such as a xenon lamp, and the rotation filter instead of the four-color LEDs 20a to 20d. Additionally, the observation target may be imaged by a monochrome imaging sensor instead of the color imaging sensor 38. In the following, only portions different from Embodiments 1A and 1B will be described, and description of substantially the same portions as those of the first embodiment will be omitted.

In an endoscope system 200 illustrated in Fig. 14, in the light source device 28, a white light source 202, a rotation filter 204, and a filter switching unit 206 are provided instead of the respective LEDs 20a to 20d of the endoscope system 10. Additionally, the imaging optical system 30b is provided with a monochrome imaging sensor 208, which is not provided with a color filter, instead of the color imaging sensor 38.

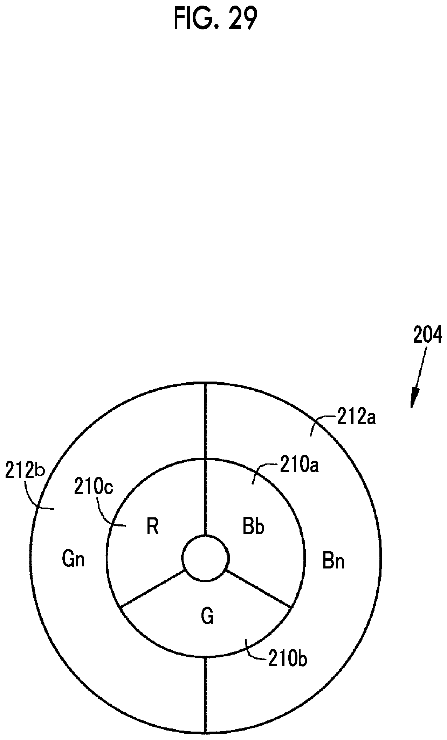

The white light source 202 is a xenon lamp, a white LED, or the like, and emits white light of which the wavelength range ranges from blue to red. The rotation filter 204 comprises a normal mode filter 210 that is provided on an inner side closest to a rotation axis thereof, and a special mode filter 212 provided outside the normal mode filter 210 (refer to Fig. 29).

The filter switching unit 206 moves the rotation filter 204 in a radial direction. Specifically, the filter switching unit 206 inserts the normal mode filter 210 into a white light path in a case where the normal mode is set by the mode switching unit 13c. Specifically, the filter switching unit 206 inserts the special mode or individual-tissue display mode filter 212 into the white light path in a case where the special mode or the individual-tissue display mode is set by the mode switching unit 13c.

As illustrated in Fig. 29, a Bb filter 210a, a G filter 210b, and an R filter 210c are provided in the circumferential direction in the normal mode filter 210. The Bb filter 210a transmits the broadband blue light Bb, which has a wavelength range of 400 to 500 nm, in the white light. The G filter 210b transmits the green light G in the white light. The R filter 210c transmits the red light R in the white light. Hence, in the normal mode, as the rotation filter 204 rotates, the broadband blue light Bb, the green light G, and the red light R are sequentially radiated toward the observation target as the normal light.

A Bn filter 212a and a Gn filter 212b are provided in the circumferential direction in the special mode or individual-tissue display mode filter 212. The Bn filter 212a transmits narrowband blue light Bn of 400 to 450 nm in the white light. The Gn filter 212b transmits narrowband green light Gn of 530 to 570 nm in the white light. Hence, in the special mode, as the rotation filter 204 rotates, the narrowband blue light and the narrowband green light are sequentially radiated toward the observation target as the special light.

In the endoscope system 200, in the normal mode, whenever the observation target is illuminated with the broadband blue light Bb, the green light G, and the red light R, the observation target is imaged by the monochrome imaging sensor 208. As a result, the Bc image signal is obtained at the time of the illumination with the broadband blue light Bb, the Gc image signal is obtained at the time of the illumination with the green light G, and the Rc image signal is obtained at the time of the illumination with the red light R. The normal image is constituted of the Bn image signal, the Gc image signal, and the Rc image signal.

In the special mode or the individual-tissue display mode, the observation target is imaged by the monochrome imaging sensor 208 whenever the observation target is illuminated with narrowband blue light Bn and the narrowband green light Gn. Accordingly, the Bn image signal is obtained at the time of the illumination with the narrowband blue light Bn, and the Gn image signal is obtained at the time of the irradiation with the narrowband green light Gn. The special image or the individual-tissue image is constituted of the Bn image signal and the Gn image signal.

### Explanation of References

- 10:: endoscope system
- 12:: endoscope
- 12a:: insertion part
- 12b:: operating part
- 12c:: bending part
- 12d:: distal end part
- 13a:: angle knob
- 13b:: still image acquisition unit
- 13c:: mode switching unit
- 13d:: zooming operating unit
- 14:: light source device
- 16:: processor device
- 18:: monitor
- 19:: console
- 20:: light source
- 20a:: V-LED
- 20b:: B-LED
- 20c:: G-LED
- 20d:: R-LED
- 22:: light source control unit
- 23:: wavelength cutoff filter
- 24:: light guide
- 30a:: illumination optical system
- 30b:: imaging optical system
- 32:: illumination lens
- 34:: objective lens
- 36:: magnifying optical system
- 36a:: zoom lens
- 36b:: lens drive unit
- 38:: imaging sensor
- 40:: CDS/AGC circuit
- 42:: A/D conversion circuit
- 50:: image signal acquisition unit
- 52:: DSP
- 54:: noise reduction unit
- 58:: image processing unit
- 60:: display control unit
- 62:: normal image processing unit
- 64:: special image processing unit
- 66:: individual-tissue image processing unit
- 70:: brightness discrimination unit
- 72:: shape discrimination unit
- 74:: tissue discrimination unit
- 76:: image generation unit
- 78:: index value calculation unit
- 90:: difference image generation unit
- 100:: endoscope system
- 104:: blue laser light source
- 106:: blue-violet laser light source
- 108:: light source control unit
- 110:: fluorescent body
- 200:: endoscope system
- 202:: white light source
- 204:: rotation filter
- 206:: filter switching unit
- 208:: imaging sensor
- 210:: normal mode filter
- 210a:: Bb filter
- 210b:: G filter
- 210c:: R filter
- 212:: special mode or individual-tissue display mode filter
- 212a:: Bn filter
- 212b:: Gn filter

## Claims

1. A processor device comprising:
an image acquisition unit that acquires images in a plurality of bands obtained by imaging an observation target including at least one tissue;
a specific calculation processing unit that performs specific calculation processing on the basis of the images in the plurality of bands to obtain a calculated image; and
a tissue discrimination unit that discriminates the tissue on the basis of a brightness value of at least one image of band among the images in the plurality of bands and the calculated image.

2. The processor device according to claim 1, further comprising:
a brightness discrimination unit that discriminates a high-brightness region where the brightness value is equal to or more than a specific threshold value, and a low-brightness region where the brightness value is less than the specific threshold value,
wherein the tissue discrimination unit discriminates the tissue among candidates of the tissue included in at least one of the high-brightness region or the low-brightness region in accordance with contents of the calculated image.

3. The processor device according to claim 2,
wherein the specific calculation processing is shape discrimination processing for discriminating a shape of the tissue, and the calculated image is an image subjected to the shape discrimination processing, and
wherein the tissue discrimination unit discriminates the tissue in the low-brightness region in accordance with the shape.

4. The processor device according to claim 2,
wherein the specific calculation processing is difference processing for combining the images in the plurality of bands to calculate a difference between the images in the plurality of bands or ratio calculation processing for combining the images in the plurality of bands to calculate a ratio of the images in the plurality of bands, and the calculated image is an image subjected to the difference processing or an image subjected to the ratio calculation processing, and
wherein the tissue discrimination unit discriminates the tissue in the low-brightness region in accordance with the difference or the ratio.

5. The processor device according to any one of claims 1 to 4,
wherein the tissue is a capillary, a crypt opening, an intervening part, a marginal crypt epithelium, an intra-epithelial papillary capillary loop (IPCL), or a dendritic blood vessel.

6. An endoscope system comprising:
the processor device according to any one of claims 1 to 5;
an individual-tissue image generation unit that generates an individual-tissue image distinguished and displayed for each tissue in accordance with a discrimination result of the tissue discrimination unit; and
a display unit that displays the individual-tissue image.

7. The endoscope system according to claim 6,
wherein a specific tissue among the tissues is displayed in an enhanced manner on the individual-tissue image.

8. The endoscope system according to claim 6,
wherein the individual-tissue image is displayed in different colors for each tissue.

9. The endoscope system according to claim 8,
wherein a plurality of tissues are respectively displayed in a switched manner in accordance with specific display timings on the individual-tissue image.

10. A processor device comprising:
an image acquisition unit that acquires images in a plurality of bands obtained by imaging an observation target including at least one tissue;
a brightness discrimination unit that discriminates a high-brightness region where a brightness value at least one image of band among the images in the plurality of bands is equal to or more than a specific threshold value, and a low-brightness region where the brightness value is less than the specific threshold value;
a shape discrimination unit that performs shape discrimination processing for discriminating a shape of the tissue on the basis of the images in the plurality of bands; and
a tissue discrimination unit that discriminates the tissue among candidates of the tissue included in at least one of the high-brightness region or the low-brightness region in accordance with the shape.

11. A method of operating a processor device comprising the steps of:
acquiring images in a plurality of bands obtained by imaging an observation target including at least one tissue, using an image acquisition unit;
performing specific calculation processing on the basis of the images in the plurality of bands to obtain a calculated image, using a specific calculation processing unit; and
discriminating the tissue on the basis of a brightness value of at least one image of band among the images in the plurality of bands and the calculated image, using a tissue discrimination unit.
